(19) Europäisches Patentamt
European Patent Office
Office européen des brevets

(11) **EP 4 389 823 A1**

(12) **EUROPEAN PATENT APPLICATION**
published in accordance with Art. 153(4) EPC

(43) Date of publication:
**26.06.2024 Bulletin 2024/26**

(21) Application number: **22858349.8**

(22) Date of filing: **05.08.2022**

(51) International Patent Classification (IPC):
**C08L 53/02** (2006.01)　　　**C08L 51/06** (2006.01)
**C08L 71/12** (2006.01)

(52) Cooperative Patent Classification (CPC):
**C08L 51/06; C08L 53/02; C08L 71/12; Y02W 30/62**

(86) International application number:
**PCT/JP2022/030123**

(87) International publication number:
**WO 2023/022028 (23.02.2023 Gazette 2023/08)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.08.2021  JP 2021134245**

(71) Applicant: **MCPP Innovation LLC
Tokyo 100-8251 (JP)**

(72) Inventor: **SUZUKI, Miyu
Tokyo 100-8251 (JP)**

(74) Representative: **Vossius & Partner
Patentanwälte Rechtsanwälte mbB
Siebertstrasse 3
81675 München (DE)**

(54) **THERMOPLASTIC ELASTOMER COMPOSITION**

(57)　A thermoplastic elastomer composition comprising the following component (A) and component (B). Component (A): a styrene-based thermoplastic elastomer having the following molecular weight distribution. The styrene-based thermoplastic elastomer has a peak top in the range of 360,000 or more and 600,000 or less in a differential molecular weight distribution diagram calculated in terms of styrene obtained by gel permeation chromatographic analysis of the styrene thermoplastic elastomer. Component (B): hydrocarbon group-grafted polypropylene

[Fig. 1]

**Description**

Technical Field

The present invention relates to a thermoplastic elastomer composition. The present invention also relates to a molded article made of the thermoplastic elastomer composition.

Background Art

Thermoplastic elastomers are soft materials with rubber elasticity and have the same moldability and recyclability as thermoplastic resins. Thermoplastic elastomers are produced without a vulcanization process. Thermoplastic elastomers have been used in a wide variety of industrial fields such as automobile parts and medical parts in recent years. Among these, as medical parts need to be sanitary, thermoplastic elastomers that do not contain vulcanizing agents or vulcanization accelerators are widely used as substitutes for vulcanized rubber.

Certain types of thermoplastic elastomer compositions have been improved to have compression set equivalent to that of vulcanized rubber. Certain types of thermoplastic elastomer compositions have been improved in liquid-leakage sealing properties when used as plugs such as cap liners, packing, and rubber plugs. As one of such thermoplastic elastomer compositions, a thermoplastic elastomer composition has been proposed that uses a modified polypropylene-based resin having a specific structure and further contains a specific amount of a block copolymer and a hydrocarbon softener for rubber (see Patent Literature 1).

Furthermore, a thermoplastic elastomer using a styrene-based elastomer having multiple peaks or peak shoulders on the relatively low molecular weight side has been proposed (see Patent Literature 2) in which a molecular weight distribution of styrene-based elastomers is focused.

[Patent Literature 1] WO 2017/150714 A1
[Patent Literature 2] JP 5703990 B

According to the inventor's detailed study, the thermoplastic elastomer composition described in above Patent Literature 1 still has insufficient compression set, as shown in Comparative Example 2 which will be described later and has not been reached the performance level required for conventional vulcanized rubber.

The thermoplastic elastomer composition described in above Patent Literature 2 also still has insufficient compression set, as shown in Comparative Examples 4 to 9 which will be described later and has not reached the performance level required for conventional vulcanized rubber.

Summary of Invention

An object of the present invention is to provide a thermoplastic elastomer composition that has both excellent compression set and moldability, and a molded article made of the thermoplastic elastomer composition.

The present inventors have discovered that a thermoplastic elastomer composition containing a styrene-based thermoplastic elastomer having a specific molecular weight distribution and a hydrocarbon group-grafted polypropylene can achieve both excellent compression set and moldability and solve the problems of the conventional technology.

The gist of the present invention resides in the following [1] to [12].

[1] A thermoplastic elastomer composition comprising the following component (A) and component (B).

Component (A): a styrene-based thermoplastic elastomer having the following molecular weight d istri bution.

The styrene-based thermoplastic elastomer has a peak top in the range of 360,000 or more and 600,000 or less in a differential molecular weight distribution diagram calculated in terms of styrene obtained by gel permeation chromatographic analysis of the styrene thermoplastic elastomer.

Component (B): hydrocarbon group-grafted polypropylene

[2] The thermoplastic elastomer composition according to [1], wherein the component (A) is a styrene-based thermoplastic elastomer having the following molecular weight distribution.

The styrene-based thermoplastic elastomer has a peak top in the range of 400,000 or more and 600,000 or less in a differential molecular weight distribution diagram calculated in terms of styrene obtained by gel permeation chromatographic analysis of the styrene thermoplastic elastomer.

[3] The thermoplastic elastomer composition according to [2], wherein the component (A) is a styrene-based thermoplastic elastomer having the following molecular weight distribution.

The styrene-based thermoplastic elastomer has a peak Lp having a peak top in the range of 100,000 or more and 400,000 or less and a peak Hp having a peak top in the range more than 400,000 and 600,000 or less in a differential molecular weight distribution diagram calculated in terms of styrene obtained by gel permeation chromatographic analysis of the styrene thermoplastic elastomer.

[4] The thermoplastic elastomer composition according to any one of [1] to [3], further comprising the following component (C).

Component (C): hydrocarbon softener for rubber

[5] The thermoplastic elastomer composition according to [4], wherein the content of the component (B) is 3% by mass or more and 30% by mass or less in a total of 100% by mass of the components (A), (B) and (C).

[6] The thermoplastic elastomer composition according to [4] or [5], wherein the content of the component (A) is 35% by mass or more and 65% by mass or less in a total of 100% by mass of the components (A) and (C).

[7] The thermoplastic elastomer composition according to any one of [1] to [6], having a compression set of 40% or less.

[8] The thermoplastic elastomer composition according to any one of [1] to [7], having a melt flow rate (230°C, load 5 kgf) of 0.5 g/10 minutes or more and 200 g/10 minutes or less.

[9] The thermoplastic elastomer composition according to any one of [1] to [8], having a Duro hardness A of 10 or more and 50 or less.

[10] The thermoplastic elastomer composition according to any one of [1] to [9], further comprising the following component (D).

Component (D): polyphenylene ether-based resin composition

[11] A molded article made of the thermoplastic elastomer composition according to any one of [1] to [10].

[12] The molded article according to [11], wherein the molded article is a rubber stopper having a needle pricking part.

Advantageous Effects of Invention

The thermoplastic elastomer composition of the present invention has both excellent compression set and moldability. Therefore, according to the thermoplastic elastomer composition of the present invention, a molded article having excellent compression set can be provided with good moldability.

The molded article of the present invention can be suitably used for rubber stoppers having a needle pricking part such as syringe gaskets, rubber stoppers for infusion bags and vial bottles, cap liners, packing, etc., which require liquid leak sealability.

Brief Description of Drawings

[Fig. 1] Figs. 1(a) and 1(b) are drawings showing an example of an embodiment of a rubber stopper having a needle pricking part, in which Fig. 1(a) is a plan view and Fig. 1(b) is a cross section along line B-B in Fig. 1(a).
[Fig. 2] Figs. 2(a) and 2(b) are drawings showing another example of the embodiment of the rubber stopper having a needle pricking part, in which Fig. 2(a) is a plan view and Fig. 2(b) is a cross section along line B-B in Fig. 2(a).
[Fig. 3] Figs. 3(a) and 3(b) are drawings showing another example of the embodiment of the rubber stopper having a needle pricking part, in which Fig. 3(a) is a plan view and Fig. 3(b) is a cross section along line B-B in Fig. 3(a).
[Fig. 4] Figs. 4(a) and 4(b) are drawings showing still another example of the embodiment of the rubber stopper having a needle pricking part, in which Fig. 4(a) is a plan view and Fig. 4(b) is a cross section along line B-B in Fig. 4(a).
[Fig. 5] Figs. 5(a) and 5(b) are drawings showing still another example of the embodiment of the rubber stopper having a needle pricking part, in which Fig. 5(a) is a plan view and Fig. 5(b) is a cross section along line B-B in Fig. 5(a).

Description of Embodiments

Embodiments of the present invention will be described in detail below.

The following description is an example of the embodiment of the present invention, ant the present invention is not limited to the following description. The present invention can be modified and practiced in any manner that is consistent with the scope of the present invention.

In this specification, when numerical values or physical property values are expressed by using "to" with preceding and following numbers, it is to be understood that the preceding and following numbers are included.

[Thermoplastic elastomer composition]

The thermoplastic elastomer composition of the present invention is characterized by containing the following component (A) and component (B).

Component (A): a styrene-based thermoplastic elastomer having the following molecular weight distribution

The styrene-based thermoplastic elastomer has a peak top in the range of 360,000 or more and 600,000 or less in a differential molecular weight distribution diagram calculated in terms of styrene obtained by gel permeation chromatographic analysis of the styrene thermoplastic elastomer.

Component (B): hydrocarbon group-grafted polypropylene

<Mechanism>

The mechanism by which the thermoplastic elastomer composition of the present invention can achieve both excellent compression set and moldability is considered to be as follows.
It is considered that a styrene-based thermoplastic elastomer having the above-mentioned unique molecular weight distribution can achieve both high moldability and low compression set.
On the other hand, a hydrocarbon group-grafted polypropylene is considered to have a low compression set because the crystal lamellae of polypropylene become stronger and plastic deformation of the matrix is less likely to occur.
Furthermore, the coexistence of the component (A) and the component (B) strongly acts as a cohesive interaction between domains and between matrices, and the lamellar structure that is the crystalline component of the component (B) is more likely to be firmly maintained. This results in excellent compression set.
In particular, when the component (A) is a styrene-based thermoplastic elastomer having the peaks Lp and Hp as described below, the component present in the peak Lp of the component (A) is moderately entangled with the component present in the peak Hp, and the viscosity of the polymer chain derived from the peak Hp component easily decreases in the molten state. This results in high moldability.
In addition, in the non-molten state, the entanglement of the polymer chains of the domain is increased by the component present in the peak Hp of component (A), resulting in low compression set.

<Component (A): Styrene-based thermoplastic elastomer>

The styrene-based thermoplastic elastomer of the component (A) has a peak top in the range of 360,000 or more and 600,000 or less in the differential molecular weight distribution diagram calculated in terms of styrene obtained by gel permeation chromatographic analysis of the styrene thermoplastic elastomer. It is preferable to have a peak top in the range of more than 400,000 and 600,000 or less, and it is more preferable to have a peak Lp having a peak top in the range of 100,000 or more and 400,000 or less, and a peak Hp having a peak top in the range of more than 400,000 and 600,000 or less.
Here, the peaks Lp and Hp do not include shoulder peaks or subpeaks having an intensity of 0.1 or less with respect to dw/dlogM of the most intense (highest) peak.
The shoulder peak generally refers to a small peak that exists on the tailing or leading of the main peak. The shoulder peak can be separated in the differential molecular weight distribution diagram of styrene-based thermoplastic elastomers by the Gauss-Newton method as a peak with its shoulder portion at the top.
The molecular weight distribution (PDI), peak top molecular weight, its dw/dlogM, the mass average molecular weight (Mw), and number average molecular weight (Mn) of the styrene-based thermoplastic elastomer described below are determined by gel permeation chromatography (hereinafter referred to as GPC). They are a polystyrene equivalent values measured under the following conditions, and the analysis range is a molecular weight of 10,000 or more.

<GPC measurement conditions>

Equipment: HLC-8220 GPC manufactured by Tosoh Corporation
Column: TSKgel Super HM-M manufactured by Tosoh Corporation
Detector: Differential refractive index detector (RI/built-in)
Solvent: Chloroform, special grade
Temperature: 40°C

Flow rate: 0.3mL/min
Injection volume: 20$\mu$L
Concentration: 0.1% by mass
Calibration sample: Monodisperse polystyrene
Calibration method: Polystyrene equivalent

The styrene-based thermoplastic elastomer of the component (A) may be a single styrene-based thermoplastic elastomer having the above mentioned molecular weight distribution, or a styrene-based thermoplastic elastomer as the component (A) having the above mentioned molecular weight distribution by mixing and using a plurality of styrene-based thermoplastic elastomers having different molecular weight distributions. However, it is preferable to manufacture a thermoplastic elastomer composition by blending a single styrene-based thermoplastic elastomer having the above mentioned molecular weight distribution, especially a single styrene-based thermoplastic elastomer having the above peak Lp and peak Hp, with the component (B) and the component (C) which will be described later. That is, by blending this single styrene-based thermoplastic elastomer with the component (B) and the component (C), which will be described later, to manufacture a thermoplastic elastomer composition, the entanglement of the component present at the peak Lp and the component present at the peak Hp of the component (A) is promoted, and the compression set tends to improve.

It is more preferable that component (A) has a peak top of peak Lp in the range of 150,000 or more and 300,000 or less, and a peak top of peak Hp in the range of 450,000 or more and 550,000 or less.

The ratio of the peak top molecular weight of peak Hp to the peak top molecular weight of peak Lp (peak top molecular weight of peak Hp/peak top molecular weight of peak Lp) is preferably 1.5 or more and 4 or less and more preferably 2 or more and 3.5 or less from the viewpoint of moldability and compression set.

The styrene-based thermoplastic elastomer may have peaks other than peak Lp and peak Hp. In such case, the peak top height other than peak Lp and peak Hp is preferably equal to or less than the lower of the peak top heights of peak Lp and peak Hp, and more preferably equal to or less than 1/2.

As the styrene-based thermoplastic elastomer of the component (A), a block copolymer selected from the group consisting of a block copolymer having at least one polymer block P derived from a vinyl aromatic compound and at least one polymer block Q derived from a conjugated diene, and a block copolymer obtained by hydrogenating the block copolymer (hydrogenated block copolymer) can be used.

The polymer block P is a polymer block of a monomer mainly consisting of a vinyl aromatic compound. The polymer block Q is a polymer block of a monomer mainly consisting of a conjugated diene. Here, "mainly" means that the content ratio in the block is 50 mol% or more.

The vinyl aromatic compounds of the monomer comprising the polymeric block P are not limited, but styrene derivatives such as styrene, $\alpha$-methylstyrene, and chloromethylstyrene are preferred. Among these, styrene is preferred as the main component. One of these may be used alone, or two or more can be used in combination. The polymer block P may contain monomers other than vinyl aromatic compounds as raw materials.

The monomers constituting the polymeric block Q are not limited, but butadiene alone, isoprene alone, or a mixture of butadiene and isoprene is preferred. One of these can be used alone, or two or more can be used in combination. The polymer block Q may contain monomers other than butadiene and isoprene as raw materials.

The polymer block Q may be a hydrogenated derivative obtained by hydrogenating the double bonds after polymerization, that is a hydrogenated block copolymer. The hydrogenation rate of the polymer block Q is not limited, but is preferably 50% or more and 100% or less by mass, and more preferably 80% or more and 100% or less by mass. By hydrogenating the polymer block Q within the above range, thermal stability and weathering stability tend to improve. The same applies to the case where the polymer block P uses a diene component as a raw material. The hydrogenation rate can be measured by $^{13}$C-NMR.

The styrene-based thermoplastic elastomer preferably has a styrene unit content of 8% or more and 45% or less by mass. When the styrene unit content of the styrene-based thermoplastic elastomer is at least the above lower limit, the bleeding out of the hydrocarbon softener for rubber from the thermoplastic elastomer composition can be suppressed. When the styrene unit content of the styrene-based thermoplastic elastomer is below the above upper limit, the hardness of the styrene-based thermoplastic elastomer can be prevented from becoming too high. The styrene unit content of the styrene-based thermoplastic elastomer is more preferably 10% or more and 40% or less by mass.

The term "styrene unit content" is used to include not only the content of styrene units, but also the content of component units in which atoms or atomic groups other than hydrogen atoms are substituted into the aromatic ring of the styrene unit. The styrene unit content can be measured by $^{13}$C-NMR.

The chemical structure of the copolymer having the polymer block P and the polymer block Q in the styrene-based thermoplastic elastomer may be linear, branched, radial, or the like. The styrene-based thermoplastic elastomer is preferably a block copolymer represented by the following formula (1) or (2).

Further, the block copolymer represented by the following formula (1) or (2) is preferably a hydrogenated derivative (hydrogenated block copolymer). When the copolymer represented by the following formula (1) or (2) is a hydrogenated block copolymer, heat resistance stability and weather resistance stability tend to be good.

$$P\text{-}(Q\text{-}P)_m \qquad (1)$$

$$(P\text{-}Q)_n \qquad (2)$$

(In the formula, P represents the polymer block P, and Q represents the polymer block Q. m represents an integer from 1 to 5. n represents an integer from 1 to 5.)

In the formula (1) or (2), m and n are preferably larger in terms of lowering the order-disorder transition temperature as a rubber-like polymer, but smaller in terms of ease of production and cost.

As block copolymer and/or hydrogenated block copolymer of styrene-based thermoplastic elastomer (hereinafter collectively referred to as "(hydrogenated) block copolymers"), the (hydrogenated) block copolymer represented by the formula (1) is more preferable than the (hydrogenated) block copolymer represented by the formula (2) because of its superior rubber elasticity.

The upper limit of the mass average molecular weight (Mw) of the styrene-based thermoplastic elastomer is not limited, but is usually 700,000 or less, preferably 600,000 or less, more preferably 500,000 or less. The lower limit of the mass average molecular weight (Mw) of styrene-based thermoplastic elastomers is not limited, but is usually 40,000 or more, preferably 60,000 or more, more preferably 100,000 or more. By controlling the mass average molecular weight of the styrene-based thermoplastic elastomer to be equal to or less than the above upper limit, moldability and appearance of the molded article can be maintained favorably. By controlling the mass average molecular weight of the styrene-based thermoplastic elastomer to be equal to or more than the above lower limit, it is possible to suppress bleeding out of the hydrocarbon softener for rubber from the thermoplastic elastomer composition and to reduce compression set.

The upper limit of the number average molecular weight (Mn) of the styrene-based thermoplastic elastomer is not limited, but is usually 600,000 or less, preferably 500,000 or less, and more preferably 400,000 or less. The lower limit of the number average molecular weight (Mn) of the styrene-based thermoplastic elastomer is not limited, but is usually 30,000 or more, preferably 50,000 or more, and more preferably 80,000 or more. By controlling the number average molecular weight of the styrene-based thermoplastic elastomer to be equal to or less than the above upper limit, moldability and appearance of the molded article can be maintained favorably. By controlling the number average molecular weight of the styrene-based thermoplastic elastomer to be equal to or more than the above lower limit, it is possible to suppress bleeding out of the hydrocarbon softener for rubber from the thermoplastic elastomer composition and to reduce compression set.

The method for producing the styrene-based thermoplastic elastomer is not particularly limited and may be any method as long as the above structure and physical properties can be obtained. For example, it can be obtained by block polymerization in an inert solvent using a lithium catalyst or the like. For the hydrogenation of the block copolymer, a known method such as hydrogenation in an inert solvent in the presence of a hydrogenation catalyst can be employed.

Among those described above, styrene-conjugated diene block copolymers and/or their hydrogenated products are preferred as styrene-based thermoplastic elastomers, and hydrogenated products in which the conjugated diene of the styrene-conjugated diene block copolymer is composed of one or more selected from isoprene and butadiene are suitable. The styrene-conjugated diene block copolymer and/or its hydrogenated product may have a polar group if necessary.

Examples of the styrene-conjugated diene block copolymer and/or its hydrogenated product include styrene-butadiene block copolymer and/or its hydrogenated product, styrene-butadiene-styrene block copolymer and/or its hydrogenated product, styrene-isoprene block copolymer and/or its hydrogenated product, styrene-isoprene-styrene block copolymer and/or its hydrogenated product, styrene-butadiene-isoprene block copolymer and/or its hydrogenated product, and styrene-isoprene-butadiene-styrene block copolymer and/or its hydrogenated product.

Examples of hydrogenated products of styrene-butadiene block copolymer include styrene-butadiene-butylene copolymer (SBB) and styrene-ethylene-butylene copolymer (SEB).

Examples of hydrogenated products of styrene-butadiene-styrene block copolymer include styrene-ethylene-butylene-styrene copolymers (SEBS).

Examples of hydrogenated products of styrene-isoprene-styrene block copolymer include styrene-ethylene-propylene-styrene copolymer (SEPS).

Examples of hydrogenated products of styrene-isoprene-butadiene-styrene block copolymer include styrene-ethylene-ethylene-propylene-styrene copolymer (SEEPS).

Among these, from the viewpoint of tensile strength and compression set, styrene-ethylene-butylene copolymer (SEB) which is a hydrogenated product of styrene-butadiene block copolymer, styrene-ethylene-butylene-styrene copolymer (SEBS) which is a hydrogenated product of styrene-butadiene-styrene block copolymer, styrene-ethylene-ethylene-propylene-styrene copolymer (SEEPS) which is a hydrogenated product of styrene-isoprene-butadiene-styrene block copolymer, and styrene-ethylene-propylene-styrene copolymer (SEPS) which is a hydrogenated product of styrene-isoprene-styrene block copolymer are preferred. These may be completely hydrogenated or partially hydrogenated.

It is also possible to use a commercially available styrene-based thermoplastic elastomer. As a commercially available product, an appropriate product can be selected and used from the "Tuftec (registered trademark)" series manufactured by Asahi Kasei Corp oration.

The styrene-based thermoplastic elastomer may be used alone or in combination of two or more different in physical properties, block structure, or hydrogenation or non-hydrogenation, etc.

<Component (B): Hydrocarbon group-grafted polypropylene>

The thermoplastic elastomer composition of the present invention contains polypropylene grafted with a hydrocarbon group as the component (B). Hydrocarbon group-grafted polypropylene is considered to provide a low compression set because the polypropylene crystal lamellae become strong, making plastic deformation of the matrix less likely to occur. Hydrocarbon group-grafted polypropylene is obtained by graft-modifying a propylene-based resin with a diene compound.

The propylene-based resin used as a raw material for the component (B) is specifically a propylene homopolymer or random copolymer, and examples thereof include crystalline polymers. As the propylene copolymer, one containing 75% by mass or more of propylene units is preferable because it maintains the characteristics of polypropylene, such as crystallinity, rigidity, and chemical resistance.

Monomers copolymerizable with propylene constituting the propylene copolymer used as a raw material for the component (B) include a -olefins having 2 or 4 to 12 carbon atoms such as ethylene, 1-butene, isobutene, 1-pentene, 3-methyl-1-butene, 1-hexene, 4-methyl-1-pentene, 3,4-dimethyl-1-butene, 1-heptene, 3-methyl-1-hexene, 1-octene, and 1-decene or the like; cyclic olefins such as cyclopentene, norbornene, and tetracyclo[6,2,11,8,13,6]-4-dodecene or the like ; dienes such as 5-methylene-2-norbornene, 5-ethylidene-2-norbornene, 1,4-hexadiene , methyl-1,4-hexadiene, and 7-methyl-1,6-octadiene or the like; vinyl monomers such as vinyl chloride, vinylidene chloride, acrylonitrile, vinyl acetate, acrylic acid, methacrylic acid, maleic acid, ethyl acrylate, butyl acrylate, methyl methacrylate, maleic anhydride, styrene, methylstyrene, vinyltoluene, and divinylbenzene or the like. These compounds may be used alone or in combination of two or more. Among these, ethylene and 1-butene are preferred because they are inexpensive and easy to handle.

Only one type of raw material propylene-based resin may be used, or two or more types may be used.

Examples of diene compounds that can be used for graft modification of the above raw material propylene-based resin include conjugated diene compounds such as butadiene, isoprene, 1,3-heptadiene, 2,3-dimethylbutadiene, and 2,5-dimethyl-2,4-hexadiene or the like. These compounds may be used alone or in any combination and ratio of two or more. Among these, butadiene and isoprene are preferred, and isoprene is particularly preferred.

Any method may be used for modification to obtain component (B). For example, it can be produced by the method described in JP2015-98542 A. Other examples include a method of irradiating the polypropylene-based resin with radiation, or a method of melt-mixing a polypropylene-based resin, a diene compound, and a radical generator. Among these, a method of melt-mixing a polypropylene-based resin, a diene compound, and a radical generator is preferred because it does not require expensive apparatus and can produce modified polypropylene at low cost.

Apparatuses for reacting a propylene-based resin, a diene compound, and a radical generator include kneading apparatuses such as rolls, co-kneaders, Banbury mixers, Brabenders, extruders such as single-screw extruders, and twin-screw extruders; horizontal agitators such as twin-axis surface renewal devises, and two-axis multi-disc devices; and a vertical agitators such as double helical ribbon agitators or the like. Among these, it is preferable to use a kneading apparatus, and an extruder is particularly preferable from the viewpoint of productivity.

There are no particular restrictions on the order or method of mixing and kneading (stirring) the propylene-based resin, the diene compound, and the radical generator. After mixing the polypropylene-based resin, the diene compound, and the radical generator, the mixture may be melt-kneaded (stirred). After melt-kneading (stirring) the propylene-based resin, the diene compound or the radical generator may be mixed simultaneously or separately, all at once or in portions. The propylene-based resin and either the diene compound or the radical generator may be melt-kneaded (stirred),and then the other of the diene compound and the radical generator may be added and melt-kneaded (stirred).

The temperature of the kneader (stirrer) is preferably 130°C or higher and 300°C or lower because the propylene-based resin melts and does not thermally decompose. The kneading (stirring) time is generally preferably 1 minute of more and 60 minutes or less.

The shape and size of the hydrocarbon group-grafted polypropylene thus obtained are not limited, and may be in the form of pellets.

The blending ratio of the diene compound to the polypropylene-based resin is not particularly limited. The blending ratio of the diene compound to 100 parts by mass of the propylene-based resin is usually 0.01 parts by mass or more, preferably 0.05 parts by mass or more, more preferably 0.1 parts by mass or more, and usually 30 parts by mass or less, preferably 10 parts by mass or less, more preferably 5 parts by mass or less.

Together with the diene compound, one or more monomers copolymerizable with the diene compound, such as vinyl chloride, vinylidene chloride, acrylonitrile, methacrylonitrile, acrylamide, methacrylamide, vinyl acetate, acrylic acid, methacrylic acid, maleic acid, maleic anhydride, acrylic acid metal salt, methacrylic acid metal salt, acrylic acid esters such as methyl acrylate, ethyl acrylate, butyl acrylate, 2-ethylhexyl acrylate, and stearyl acrylate or the like, methacrylic acid esters such as methyl methacrylate, ethyl methacrylate, butyl methacrylate, 2-ethylhexyl methacrylate, and stearyl methacrylate or the like, may be used.

The radical generator is not particularly limited, but peroxides and azo compounds or the like can be used. Specific examples thereof include diacyl peroxides such as diperoxide and dilauroyl peroxide or the like, dialkyl peroxides such as dicumyl peroxide, tert-butylcumyl peroxide, di-tert-butyl peroxide, 2,5-dimethyl-2,5-di(tert-butylperoxy)hexyne-3, $\alpha,\alpha'$-bis(tert-butylperoxy-m-isopropyl)benzene, and 2,5-dimethyl-2,5-di(tert-butylperoxy)hexane or the like, alkyl peroxy esters such as tert-butyl peroxybenzoate, tert-butyl peroxy isobutyrate, tert-butyl peroxy pivalate, and cumyl peroxy pivalate or the like, peroxy carbonates such as tert-butyl peroxy isopropyl carbonate, tert-butylperoxy 2-ethylhexyl carbonate, and tert-amylperoxyisopropyl carbonate or the like, peroxyketals such as 1,1-bis(tert-butylperoxy) 3,3,5-trimethylcyclohexane, 1,1-bis(tert-butylperoxy)cyclohexane, n-butyl 4,4-bis(tert-butylperoxy)valerate, and 2,2-bis(tert-butylperoxy) butane or the like, azo compounds such as azobisisobutyronitrile, and dimethyl azoisobutyrate or the like.

These radical generators can be appropriately selected depending on the type and MFR of the raw material polypropylene-based resin, the type of the diene compound, and reaction conditions or the like. The radical generator may be used alone or in any combination and ratio of two or more.

The amount of the radical generator to be added is not particularly limited. The amount of the radical generator to be added is usually 0.001 pats by mass or more and 20 parts by mass or less, preferably 0.005 parts by mass or more and 10 parts by mass or less, more preferably 0.01 parts by mass or more and 5 parts by mass or less, and even more preferably 0.05 parts by mass or more and 4 parts by mass or less, with respect to 100 parts by mass of the polypropylene-based resin.

The melt flow rate (MFR) of the component (B) is preferably 1.0 g/10 minutes or more, more preferably 2.0 g/10 minutes or more, and preferably 200 g/10 minutes or less, more preferably 100 g/10 minutes or less. When the MFR of the component (B) is within the above range, a molded article made of the thermoplastic elastomer composition of the present invention tends to have excellent compression set and appearance.

The MFR of the component (B) is a value measured by method according to ISO 1133-1 (2011) or JIS K7210-1 under the conditions of temperature of 230°C, load of 2.16 kg, and 10 minutes.

The component (B) can be obtained as a commercial product. As a commercially available product, an appropriate one can be selected and used from the "PP processability improvement material" series manufactured by Kaneka Corporation and the "Waymax (registered trademark)" series manufactured by Japan Polypropylene Corporation, or the like.

Only one type of the component (B) may be used, or two or more types of components having different physical properties and structures or the like may be used as a mixture.

<Component (C): Hydrocarbon softener for rubber>

The thermoplastic elastomer composition of the present invention may contain a hydrocarbon softener for rubber as the component (C).

As the hydrocarbon softener for rubber as the component (C), process oil such as hydrocarbon oil is used. As the hydrocarbon oil, paraffinic, naphthenic, and other process oils are used. Among these, paraffinic process oil is preferred because it effectively softens the thermoplastic elastomer composition of the present invention.

A kinematic viscosity at 40°C of the hydrocarbon softener for rubber is not particularly limited. The kinematic viscosity is preferably 20 centistokes or more and more preferably 50 centistokes or more from the viewpoint of hygiene of the eluted fine particles and bleed-out. The kinematic viscosity is preferably 800 centistokes or less and more preferably 600 centistokes or less from the viewpoint of moldability.

A flash point (COC method) of the hydrocarbon softener for rubber is preferably 200°C or higher and more preferably 250°C or higher.

It is also possible to use a commercially available product as the hydrocarbon softener for rubber as the component (C). Commercially available products include the "Nisseki Polybutene (registered trademark)" HV series manufactured by ENEOS Corporation, the "Diana (registered trademark) process oil" PW series manufactured by Idemitsu Kosan Co., Ltd., and the "Lucant (registered trademark)" series manufactured by Mitsui Chemicals, Inc. These can be appropriately selected and used.

The hydrocarbon softener for rubber of the component (C) may be used alone or in combination of two or more.

<Content>

The thermoplastic elastomer composition of the present invention preferably contains the component (A), a styrene-based thermoplastic elastomer, as a main component. The term "main component" as used herein means that the proportion (mass) of the styrene-based thermoplastic elastomer in the entire composition is the largest.

From the viewpoint of rubber elasticity, the content of the component (A) in the thermoplastic elastomer composition of the present invention is preferably 10% by mass or more, more preferably 20% by mass or more, and even more preferably 30% by mass or more based on the total of 100% by mass of the component (A), the component (B), and the optionally used component (C). On the other hand, from the viewpoint of heat resistance, the content of the component (A) is preferably 85% by mass or less, more preferably 75% by mass or less, and even more preferably 60% by mass or less.

The content of the component (B) is preferably 3% by mass or more, more preferably 5% by mass or more, and even more preferably 8% by mass or more based on the total of 100% by mass of the component (A), the component (B), and the optionally used component (C). When it is above this lower limit, the effect of the diene compound used for graft modification of the component (B) is maximized, and low compression set tends to be easily obtained. On the other hand, the upper limit of the content of the component (B) is preferably 30% by mass or less, more preferably 25% by mass or less, and even more preferably 20% by mass or less. When it is less than this upper limit, the molded article becomes moderately soft, and therefore a low compression set tends to be easily obtained.

When the thermoplastic elastomer composition of the present invention contains the component (C), from the viewpoint of flexibility and moldability of the molded article, the content of the component (C) is preferably 10% by mass or more, more preferably 20% by mass or more, and even more preferably 30% by mass or more based on the total of 100% by mass of the components (A), (B) and (C). On the other hand, from the viewpoint of oil bleeding, the upper limit of the content of the component (C) is preferably 85% by mass or less, more preferably 75% by mass or less, and even more preferably 60% by mass or less.

When the thermoplastic elastomer composition of the present invention contains the component (C), the content of the component (A) is preferably 10% by mass or more, more preferably 20% by mass or more, even more preferably 30% by mass or more, and particularly preferably 35% by mass or more based on the total of 100% by mass of the component (A) and the component (C) from the viewpoint of oil bleeding. On the other hand, from the viewpoint of fluidity, the content of the component (A) is preferably 85% by mass or less, more preferably 80% by mass or less, even more preferably 75% by mass or less, and particularly preferably 65% by mass or less.

<Component (D): Polyphenylene ether-based resin composition>

The thermoplastic elastomer composition of the present invention may contain a polyphenylene ether-based resin composition as the component (D).

The polyphenylene ether-based resin composition refers to one whose main component is polyphenylene ether-based resin. The term "main component" as used herein means that the proportion of the amount (mass) of the polyphenylene ether-based resin in the entire composition is the largest.

Known polyphenylene ether-based resins can be used as the polyphenylene ether-based resin contained in the polyphenylene ether-based resin composition. Specific examples include poly(2,6-dimethyl-1,4-phenylene ether), poly(2-methyl-6ethyl-1,4-phenylene ether), poly(2,6-diphenyl-1,4 - phenylene ether), poly(2-methyl-6-phenyl-1,4-phenylene ether), and poly(2,6-dichloro-1,4-phenylene ether) or the like.

Polyphenylene ether-based resin compositions also include polyphenylene ether copolymers such as copolymers of 2,6-dimethylphenol and other phenols (for example, 2,3,6-trimethylphenol and 2-methyl-6-butylphenol), and polymer alloys with polystyrene.

Among them, poly(2,6-dimethyl-1,4-phenylene ether), and copolymer of 2,6-dimethylphenol and 2,3,6-trimethylphenol are preferred, and poly(2,6-dimethyl-1,4-phenylene ether) is particularly preferred.

From the viewpoint of compression set, the polyphenylene ether-based resin composition of the component (D) is preferably blended in an amount of 1 part by mass or more and 20 parts by mass or less based on the total of 100 parts by mass of the component (A), the component (B), and the optionally used component (C).

Commercially available products of the component (D) include, for example, "lupiace (registered trademark)" manufactured by Mitsubishi Engineering-Plastics Corporation and "Xyron (registered trademark)" manufactured by Asahi Kasei Corporation.

<Other Component>

The thermoplastic elastomer composition of the preferred embodiment of the present invention may contain other components other than the above-mentioned components (A) to (D) as necessary, as long as the object of the present invention is not impaired.

Other components include various fillers such as talc and calcium carbonate, various antiblocking agents, heat stabilizers, antioxidants, lubricants, crystal nucleating agents, coloring agents, and resins other than the components (A), (B), and (D).

For examples of antioxidants, phenolic antioxidants such as tetrakis[3-(3',5'-di-t-butyl-4'-hydroxyphenyl)propionic acid]pentaerythritol and 4,4',4"-[(2,4,6-trimethyl-1,3,5-benzentriyl)tris(methylene)]tris[2,6-bis(1,1-dimethylethyl) phenol, phosphorus processing stabilizers such as tris(2,4-di-t-butylphenyl) phosphite, and hydroxylamine processing heat stabilizers such as oxidation products of alkylamines made from reduced beef tallow are preferred.

When using an antioxidant, the blending amount is preferably 0.01% by mass or more and 1.0% by mass or less and more preferably 0.05% by mass or more and 0.5% by mass or less based on the total of 100% by mass of the component (A), the component (B), and the optionally used component (C).

Resins other than the components (A), (B), and (D) that may be contained in the thermoplastic elastomer composition of the preferred embodiment of the present invention include styrene-based elastomers other than the component (A), polyolefin resins other than the component (B), polyester resins, polyamide resins, acrylic resins, polycarbonate resins, polyvinyl chloride resins, polystyrene resins, liquid crystal resins, various elastomers (excluding those falling under the component (A)), and the like. The other resins mentioned above may contain only one type or two or more types.

When the thermoplastic elastomer composition of the preferred embodiment of the present invention contains resins other than the components (A), (B), and (D), in order to fully obtain the effect of containing the component (A), the component (B), and the optionally used component (C), the content of the other resins is preferably 30 parts by mass or less, more preferably 20 parts by mass or less, and even more preferably 10 parts by mass or less based on a total of 100 parts by mass of the component (A), the component (B), and the optionally used component (C).

<Method for producing the thermoplastic elastomer composition>

The thermoplastic elastomer composition of the preferred embodiment of the present invention can be obtained as a solid material such as a pellet by mechanically mixing the above-mentioned component (A), component (B) and the optionally used component (C), component (D), and other components using a known method such as a Henschel mixer, a V-blender, or a tumbler blender, and then mechanically melt-kneading by a known method and extruding through a die. For the mechanical melt-kneading, general melt-kneaders such as a Banbury mixer, various kneaders, and a single-screw or twin-screw extruder can be used.

<Physical properties>

The thermoplastic elastomer composition of the present invention has both excellent compression set and moldability by containing the component (A) and the component (B), and the optionally used component (C) and component (D). The thermoplastic elastomer composition of the present invention preferably has the following physical properties.

(Melt flow rate (MFR))

In the present invention, the melt flow rate (MFR) measured at 230°C and a load of 2.16 kgf (21 N) or 5 kgf (49 N) with reference to ISO 1133-1 (2011) is used as an indicator of a moldability of the thermoplastic elastomer composition.

In order to obtain excellent moldability, it is preferable that the melt flow rate (5 kgf) of the thermoplastic elastomer composition of the present invention is 0.5 g/10 minutes or more and 200 g/10 minutes or less. When the MFR of the thermoplastic elastomer composition is 0.5 g/10 minutes or more, the fluidity tends to be good. It is preferable from the viewpoint of heat resistance that the MFR of the thermoplastic elastomer composition is 200 g/10 minutes or less. From the viewpoint of fluidity, the MFR of the thermoplastic elastomer composition is more preferably 1 g/10 minutes or more. From the viewpoint of heat resistance, the MFR of the thermoplastic elastomer composition is more preferably 150 g/10 minutes or less, and even more preferably 100 g/10 minutes or less.

(Compression set)

The thermoplastic elastomer composition of the present invention preferably has a compression set (recovery rate) of 40% or less, more preferably 35% or less, and even more preferably 30% or less, as determined as follows. Since the compression set is 40% or less, it has recovery properties close to those of vulcanized rubber, and is suitable for liquid-leakage resistance and sealing properties.

Referring to ISO815-1, six test pieces having a diameter of 29 mm obtained by punching out a plate obtained by injection molding the thermoplastic elastomer composition of the present invention are stacked and the original thickness ($t_0$) is measured. Thereafter, it is compressed by 25% using a compression device and a spacer (thickness $t_1$), and heat treatment is performed in a gear oven maintained at 70°C for 22 hours. Thereafter, immediately after taking it out of the oven, the test piece is taken out from the compression device under an environment of 23°C and 50% RH and released from compression, and its thickness ($t_2$) is measured 30 minutes later.

The compression set is calculated according to the formula below.

## [Math. 1]

$$CS = \frac{t_0 - t_2}{t_0 - t_1} \times 100$$

CS: Compression set (%)
$t_0$: Original thickness of test piece (mm)
$t_1$: Spacer thickness (mm)
$t_2$: Thickness (mm) after 30 minutes after taking out the test piece from the compression device

(Flexibility (Duro hardness A))

The thermoplastic elastomer composition of the present invention preferably has a hardness of 10 or more and 50 or less when measured using Duro hardness A (type A durometer) after 15 seconds by stacking three plates obtained by injection molding with reference to ISO 7619-1. When the Duro hardness A of the thermoplastic elastomer composition is 50 or less, compression set tends to be good. When the Duro hardness A of the thermoplastic elastomer composition is 10 or more, it is preferred for ease of assembly. From the viewpoint of assemblability, the Duro hardness A of the thermoplastic elastomer composition is more preferably 15 or more, and even more preferably 20 or more. From the viewpoint of liquid-leakage resistance and compression set, the Duro hardness A of the thermoplastic elastomer composition is more preferably 40 or less, even more preferably 35 or less, and particularly preferably 30 or less.

From the viewpoint of liquid-leakage resistance, the thermoplastic elastomer composition of the present invention preferably has a compression set (recovery rate) of 40% or less and a duro hardness A of 40 or less, more preferably has a compression set (recovery rate) of 35% or less and a duro hardness A of 35 or less, even more preferably has a compression set (recovery rate) of 30% or less and a duro hardness A of 35 or less, and particularly preferably has a compression set (recovery rate) of 30% or less and the duro hardness A of 30 or less.

[Molded article of thermoplastic elastomer composition]

The thermoplastic elastomer composition of the present invention can be used as a molded article by various molding methods. In particular, it is preferable to form a molded article by injection molding the thermoplastic elastomer composition of the present invention. The molding conditions for injection molding are as follows.

The molding temperature when injection molding the thermoplastic elastomer composition is usually 150°C or higher and 300°C or lower, and preferably 180°C or higher and 280°C or lower. The injection pressure is usually 5MPa or more and 100MPa or less, and preferably 10MPa or more and 80MPa or less. The mold temperature is usually 0°C or higher and 80°C or lower, and preferably 20°C or higher and 60°C or lower.

[Uses]

The thermoplastic elastomer composition of the present invention can be applied to rubber stoppers having needle pricking part such as syringe gaskets, infusion bag rubber stoppers, rubber stoppers for vials, etc. cap liners, and packing or the like, which require liquid-leakage sealing properties. Its uses are not limited to medical use. For example, it is suitably used for non-medical syringes, droppers, water pitchers, bottle pumps, syringe-type feeders, gaskets for water guns, gaskets for cooking utensils, exterior moldings for automobiles, window wipers for automobiles, and the like.

[Rubber stopper having needle pricking part]

As mentioned above, the molded article of the present invention made of the thermoplastic elastomer composition of the present invention has excellent compression set and excellent liquid-leakage sealing properties, and is therefore particularly useful as a rubber stopper having a needle pricking part.

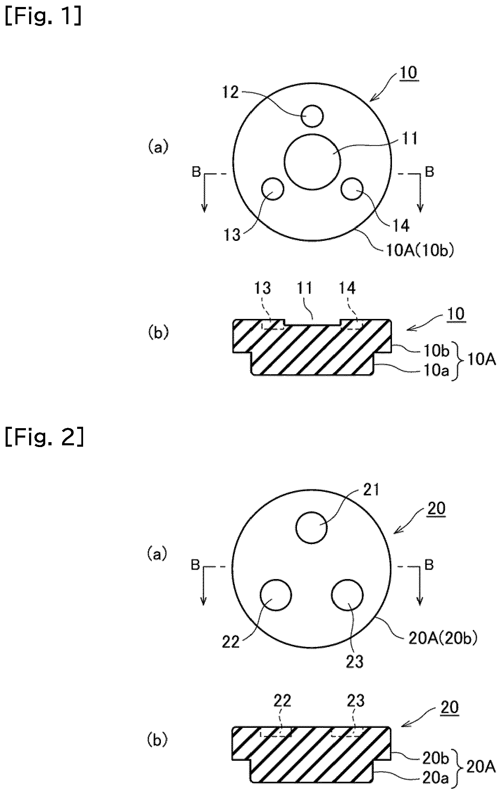

A rubber stopper having a needle pricking part as an embodiment of the molded article of the present invention will be described below with reference to Figs. 1 to 5.

However, the rubber stopper according to the present invention is not limited to those shown in Figs. 1 to 5.

The rubber stopper 10 shown in Fig. 1 has a base body 10A and needle pricking parts 11, 12, 13, and 14. The base body 10A includes a base part (leg part) 10a that is inserted into the opening of a vial or the like, and a lid part 10b. The needle pricking parts 11 to 14 are provided as recesses on the upper surface of the lid part 10b.

In this rubber stopper 10, the needle pricking parts are composed of a needle pricking part 11 having a large diameter and needle pricking parts 12 to 14 having a small diameter provided at equal intervals around the needle pricking part 11.

In this way, by having the needle pricking part 11 and the needle pricking parts 12 to 14 with different diameters, it is possible to insert needles with different diameters. For example, a needle made of plastic and having a large diameter of an infusion set can be inserted into the large diameter of needle pricking part 11 . The small diameter needle pricking parts 12 to 14 can be used for mixing for injecting a medicinal solution or to be inserted a needle made of metal having a small diameter.

The rubber stopper 20 shown in Fig. 2 has the same configuration as the rubber stopper 10 shown in Fig. 1, except that needle pricking parts 21-23 having an equal diameter are provided on the upper surface of the lid part 20b of the base body 20A at equal intervals. In Fig. 2, 20a is the base part (leg part).

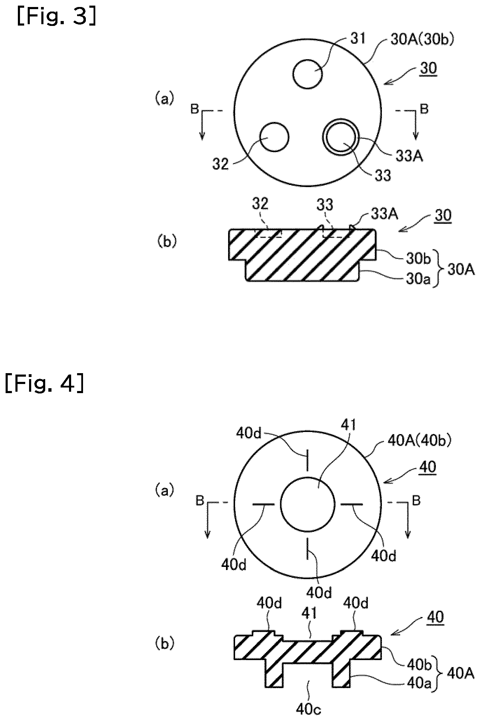

The rubber stopper 30 shown in Fig. 3 has the same configuration as the rubber stopper 20 shown in Fig. 2, except that a convex portion 33A is provided around the peripheral edge of the needle pricking part 33 among the needle pricking parts 31 to 33. In Fig. 3, 30A is the base body, 30a is the base part (leg part) and 30b is the lid part. By providing a convex portion 33A on the peripheral edge of the needle pricking part 33 like this rubber stopper 30, it becomes easy to distinguish it from other needle pricking parts 31 and 32, and it can be used as a mark for injecting the desired drug solution.

The rubber stopper 40 shown in Fig. 4 is provided with a needle pricking part 41 and four convex stripes 40d on the upper surface of the lid part 40b of the base body 40A. A recess 40c is formed in the center of the base part (leg part) 40a of the base body 40A. In this rubber stopper 40, a recess 40c is formed in the base part (leg part) 40a, and the base part (leg part) 40a is annular, so that it can be easily inserted into the mouth of a vial or the like. In addition, in this rubber stopper 40, in order to identify the rubber stopper 40, etc., the needle pricking part 41 is provided in a recessed manner in the center of the lid part 40b, and four convex stripes 40d extending in the radial direction at 90° intervals around the needle pricking part are provided.

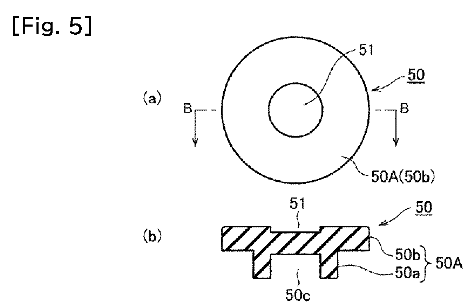

The rubber stopper 50 shown in Fig. 5 has the same configuration as the rubber stopper 40 shown in Fig. 4, except that there is no convex stripe and only a needle pricking part 51 is provided in the center of the lid part 50b. 50A is a base body comprising a base part (leg part) 50a in which a recess 50c is formed and a lid part 50b.

EXAMPLES

Now, specific embodiments of the present invention will be described in further detail with reference to examples. The present invention is not limited to the following examples as long as the present invention is within the gist thereof.

The values of various production conditions and evaluation results in the following examples are to be understood as preferred upper or lower limit values of embodiments of the present invention, and preferred ranges may be a range defined by a combination of the upper or lower limit value and a value of an example described below or by a combination of values of examples described below.

[Evaluation method]

In the following, the physical properties and characteristics of the styrene-based thermoplastic elastomer, thermoplastic elastomer composition, and molded product thereof were evaluated by the following method.

(1) Mass average molecular weight, number average molecular weight, and molecular weight distribution of the styrene-based thermoplastic elastomer of the component (A).

The molecular weights were measured by gel permeation chromatography (GPC) under the following conditions, converted to polystyrene, and a differential molecular weight distribution diagram was prepared. The analysis range was defined as a molecular weight of 10,000 or more.

<GPC measurement conditions>

Equipment: HLC-8220 GPC manufactured by Tosoh Corporation
Column: TSKgel Super HM-M manufactured by Tosoh Corporation
Detector: Differential refractive index detector (RI/built-in)
Solvent: Chloroform, special grade
Temperature: 40°C
Flow rate: 0.3mL/min
Injection volume: $20\mu$L
Concentration: 0.1% by mass
Calibration sample: Monodisperse polystyrene
Calibration method: Polystyrene equivalent

(2) Melt flow rate (MFR)

The MFR of the thermoplastic elastomer composition was measured at 230°C and a load of 2.16 kgf (21N) or 5 kgf (49N) referring to ISO 1133-1 (2011).

(3) Method of preparing a test piece (a thermoplastic elastomer composition sheets)

The pellets of the thermoplastic elastomer composition were supplied to an in-line screw type injection molding machine (IS130GN manufactured by Toshiba Machine Co., Ltd.), and injection molding was carried out under the conditions for a cylinder temperature of 190°C or higher and 210°C or lower (However, in Example 4, since d-1 was used, the cylinder temperature was 220°C or higher and 270°C or lower.), a mold temperature of 30°C or higher and 50°C or lower, an injection time of 2 seconds or more and 10 seconds or less, and a cooling time of 10 seconds or more and 40 seconds or less to obtain a sheet of the thermoplastic elastomer composition having a wall thickness of 2 mm.

(4) Evaluation of molded articles

(4-1) Compression set

Referring to ISO815-1, six test pieces having a diameter of 29 mm obtained by punching out a sheet obtained by injection molding the thermoplastic elastomer composition were stacked and the original thickness ($t_0$) was measured. Thereafter, it was compressed by 25% using a compression device and a spacer (thickness $t_1$), and heat treatment was performed in a gear oven maintained at 70°C for 22 hours. Thereafter, immediately after taking it out of the oven, the test piece was taken out from the compression device under an environment of 23°C and 50% RH and released from compression, and its thickness ($t_2$) was measured 30 minutes later. The compression set was calculated according to the formula below.

[Math. 2]

$$CS = \frac{t_0 - t_2}{t_0 - t_1} \times 100$$

CS: Compression set (%)
$t_0$: Original thickness of test piece (mm)
$t_1$: Spacer thickness (mm)
$t_2$: Thickness (mm) after 30 minutes after taking out the test piece from the compression device

(4-2) Flexibility (Duro hardness A)

Referring to ISO 7619-1, three sheets obtained by injection molding were stacked and the hardness was measured after 15 seconds using Duro hardness A (type A durometer).

[Raw Materials]

The raw materials used in the following Examples and Comparative Examples are shown below.

Component (A): Styrene-based thermoplastic elastomer

The molecular weights of the peak Lp and peak Hp of the component (A) below are as shown in Table 1.
a-1: Hydrogenated product of styrene-butadiene copolymer (styrene-ethylene-butylene-styrene copolymer (SEBS) and/or styrene-ethylene-butylene copolymer (SEB))

Product name: Tuftec (registered trademark) N527 manufactured by Asahi Kasei Corporation
Styrene unit content: 21.3% by mass
1,4-microstructure ratio of butadiene: 29.6% by mass
Mass average molecular weight (Mw): 395,000
Number average molecular weight (Mn): 210,000
Molecular weight distribution (PDI): 1.9

a-2: Styrene-ethylene-butylene-styrene copolymer (SEBS)

Product name: Tuftec (registered trademark) N504 manufactured by Asahi Kasei Corporation
Mass average molecular weight (Mw): 236,000
Number average molecular weight (Mn): 210,000
Molecular weight distribution (PDI): 1.1

a-3: Styrene-ethylene-ethylene-propylene-styrene copolymer (SEEPS)

Product name: Septon (registered trademark) 4077 manufactured by Kuraray Co., Ltd.
Mass average molecular weight (Mw): 363,000
Number average molecular weight (Mn): 270,000
Molecular weight distribution (PDI): 1.3

a-4: Styrene-ethylene-ethylene-propylene-styrene copolymer (SEEPS)

Product name: Septon (registered trademark) 4099 manufactured by Kuraray Co., Ltd.
Mass average molecular weight (Mw): 425,000
Number average molecular weight (Mn): 300,000
Molecular weight distribution (PDI): 1.4

a-5: Styrene-ethylene-ethylene-propylene-styrene copolymer (SEEPS)

Product name: Septon (registered trademark) J3341 manufactured by Kuraray Co., Ltd.
Mass average molecular weight (Mw): 350,000
Number average molecular weight (Mn): 220,000
Molecular weight distribution (PDI): 1.6

a-6: Styrene-ethylene-butylene-styrene copolymer (SEBS)

Product name: Kraton (registered trademark) G1633EU manufactured by Kraton Corporation
Mass average molecular weight (Mw): 394,000
Number average molecular weight (Mn): 310,000
Molecular weight distribution (PDI): 1.3

a-7: Styrene-ethylene-butylene-styrene copolymer (SEBS)

Product name: Kraton (registered trademark) G1654HU manufactured by Kraton Corporation
Mass average molecular weight (Mw): 168,000
Number average molecular weight (Mn): 140,000
Molecular weight distribution (PDI): 1.2

a-8: Hydrogenated product of styrene-isoprene-styrene block copolymer (vinyl SEPS)

Product name: Septon (registered trademark) 7135R manufactured by Kuraray Co., Ltd.
Mass average molecular weight (Mw): 279,000
Number average molecular weight (Mn): 210,000
Molecular weight distribution (PDI): 1.3

a-9: Styrene-isobutylene-styrene block copolymer (SIBS)

Product name: SIBSTAR (registered trademark) 103T manufactured by Kaneka Corporation
Mass average molecular weight (Mw): 132,000
Number average molecular weight (Mn): 89,000
Molecular weight distribution (PDI): 1.5

Component (B): Hydrocarbon group-grafted polypropylene

b-1: Modified polypropylene in which isoprene is grafted onto propylene homopolymer

PP processing improvement material manufactured by Kaneka Corporation
MFR (230°C, 2.16kg): 56g/10min

b-2: Modified polypropylene in which isoprene is grafted onto propylene homopolymer

PP processing improvement material manufactured by Kaneka Corporation
MFR (230°C, 2.16kg): 7g/10min

Component (X): Other resin

x-1: Propylene homopolymer

Product name: Novatec PP (registered trademark) MA1Q manufactured by Japan Polypropylene Corporation
MFR (230°C, 2.16kg): 22g/10min

x-2: Propylene homopolymer

Product name: Novatec PP (registered trademark) FA3KM manufactured by Japan Polypropylene Corporation
MFR (230°C, 2.16kg): 12g/10min

x-3: High density polyethylene

Product name: Creolex (registered trademark) T701A manufactured by Asahi Kasei Corporation
MFR (190°C, 2.16kgf): 12g/10min

Component (C): Hydrocarbon softener for rubber (hydrocarbon oil)

c-1: Product name: Diana (registered trademark) process oil PW90 manufactured by Idemitsu Kosan Co., Ltd.
c-2: Product name: Nisseki Polybutene (registered trademark) HV300 manufactured by ENEOS Corporation,

Component (D): Polyphenylene ether-based resin composition

d-1: Product name: lupiace (registered trademark) PX100F manufactured by Mitsubishi Engineering-Plastics Corporation

[Example 1]

<Production of pellets of a thermoplastic elastomer composition>

45.0 parts by mass of a-1 the styrene-based thermoplastic elastomer as component (A), 10.0 parts by mass of b-1 the hydrocarbon group-grafted polypropylene as component (B), and 45.0 parts by mass of c-1 hydrocarbon softener for rubber as component (C) were mixed to obtain a mixture. To 100 parts by mass of the obtained mixture, 0.1 part by mass of tetrakis[3-(3',5'-di-t-butyl-4'-hydroxyphenyl)propionic acid]pentaerythritol (manufactured by Adeka Corporation, product name: Adekastab (registered trademark) AO60) was added as an antioxidant, and the mixture was fed into a co-directional twin-screw extruder ("TEM26-SS" manufactured by Toshiba Machine Co., Ltd., cylinder diameter 26 mm) at a rate of 10 kg/hour or more and 20kg/hour or less. The mixture was melt-kneaded at 180°C or higher and 220°C or lower, and extruded into a strand from a die. Thereafter, it was cut to obtain a thermoplastic elastomer composition in the form of pellets. This thermoplastic elastomer composition was evaluated according to (2) to (4) above. The results are shown in Table 1.

[Examples 2-3, 5-12 and Comparative Examples 1-12]

A mixture was obtained in the same manner as in Example 1, except that the formulation was changed as shown in Tables 1 and 2, and an antioxidant was added and kneaded in the same manner to obtain a pellet-shaped thermoplastic elastomer composition. Then, evaluation was performed in the same manner as in Example 1. The results are shown in Tables 1 and 2.

[Example 4]

A mixture was obtained in the same manner as in Example 1, except that the formulation was changed as shown in Table 1. To 100 parts by mass of the obtained mixture, 0.1 part by mass of tetrakis[3-(3',5'-di-t-butyl-4'-hydroxyphenyl)propionic acid] pentaerythritol (manufactured by Adeka Corporation, product name: Adekastab (registered trademark) AO60) was added as an antioxidant was added and the mixture was fed into a co-directional twin-screw extruder ("TEM26-SS" manufactured by Toshiba Machine Co., Ltd., cylinder diameter 26 mm) at a rate of 10 kg/hour or more and 20kg/hour or less. The mixture was melt-kneaded at 230°C or higher and 270°C or lower, and extruded into a strand from a die. Thereafter, it was cut to obtain a thermoplastic elastomer composition in the form of pellets. Then, evaluation was performed in the same manner as in Example 1. The result is shown in Table 1.

[Table 1]

<Tablel 1>

| | | Example 1 | Example 2 | Example 3 | Example 4 | Example 5 | Example 6 | Example 7 | Example 8 | Example 9 | Example 10 | Example 11 | Example 12 |
|---|---|---|---|---|---|---|---|---|---|---|---|---|---|
| Composition formulation (parts by mass) | Component (A) a-1 | 45.0 | 34.8 | 36.0 | 36.0 | 45.0 | 46.5 | 30.0 | 30.0 | 30.0 | 45.0 | --- | --- |
| | a-2 | --- | --- | --- | --- | --- | --- | --- | --- | --- | --- | --- | --- |
| | a-3 | --- | --- | --- | --- | --- | --- | --- | --- | --- | --- | 45.0 | --- |
| | a-4 | --- | --- | --- | --- | --- | --- | --- | --- | --- | --- | --- | 45.0 |
| | a-5 | --- | --- | --- | --- | --- | --- | --- | --- | --- | --- | --- | --- |
| | a-6 | --- | --- | --- | --- | --- | --- | --- | --- | --- | --- | --- | --- |
| | a-7 | --- | --- | --- | --- | --- | --- | --- | --- | --- | --- | --- | --- |
| | a-8 | --- | --- | --- | --- | --- | --- | 15.0 | 15.0 | 10.0 | --- | --- | --- |
| | a-9 | --- | --- | --- | --- | --- | --- | --- | --- | 5.0 | --- | --- | --- |
| Component (B) | b-1 | 10.0 | 13.0 | 10.0 | 10.0 | 10.0 | 7.0 | 10.0 | 10.0 | 10.0 | --- | 10.0 | 10.0 |
| | b-2 | --- | --- | --- | --- | --- | --- | --- | --- | --- | 10.0 | --- | --- |
| Component (X) | x-1 | --- | --- | --- | --- | --- | --- | --- | --- | --- | --- | --- | --- |
| | x-2 | --- | --- | --- | --- | --- | --- | --- | --- | --- | --- | --- | --- |
| | x-3 | --- | --- | --- | --- | --- | --- | --- | --- | --- | --- | --- | --- |
| Component (C) | c-1 | 45.0 | 52.2 | 54.0 | 54.0 | --- | 46.5 | 45.0 | --- | --- | 45.0 | 45.0 | 45.0 |
| | c-2 | --- | --- | --- | --- | 45.0 | --- | --- | 45.0 | 45.0 | --- | --- | --- |
| Component (D) | d-1 | --- | --- | --- | 10.0 | --- | --- | --- | --- | --- | --- | --- | --- |

(continued)

| | | | Example 1 | Example 2 | Example 3 | Example 4 | Example 5 | Example 6 | Example 7 | Example 8 | Example 9 | Example 10 | Example 11 | Example 12 |
|---|---|---|---|---|---|---|---|---|---|---|---|---|---|---|
| Molecular weight of peak top of component (A) | Other peak | | --- | --- | --- | --- | --- | --- | --- | --- | 95,000 | --- | --- | --- |
| | Peak Lp | | --- | --- | --- | --- | --- | --- | --- | --- | 129,000 | --- | --- | --- |
| | | | 177,000 | 177,000 | 177,000 | 177,000 | 177,000 | 177,000 | 177,000 | 177,000 | 177,000 | 177,000 | --- | --- |
| | | | --- | --- | --- | --- | --- | --- | 301,000 | 301,000 | 301,000 | --- | 379,000 | --- |
| | Peak Hp | | 499,000 | 499,000 | 499,000 | 499,000 | 499,000 | 499,000 | 499,000 | 499,000 | 499,000 | 499,000 | --- | 457,000 |
| (A)/((A)+(C)) ※ | | | 50 | 40 | 40 | 40 | 50 | 50 | 50 | 50 | 50 | 50 | 50 | 50 |
| Evaluation results | MFR | 230°C, 2.18kpf (g/10min.) | --- | --- | --- | --- | --- | --- | --- | --- | --- | --- | --- | --- |
| | | 230°C, 5kgF (g/10min.) | 3 | 60 | 36 | 11 | 3 | 2 | 5 | 12 | 6 | 1 | 0.5 | 0.9 |
| | Comression set | 70°C, 22h (%) | 27 | 26 | 27 | 24 | 26 | 31 | 30 | 30 | 32 | 30 | 33 | 34 |
| | Duro hardness A | 15s (-) | 31 | 30 | 25 | 27 | 37 | 26 | 44 | 47 | 48 | 35 | 45 | 46 |

※Content of Component(A) relative to the total of component (A) and component (c) (% by mass)

[Table 2]

<Table2>

| Composition formulation (parts by mass) | Component(A) | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 | Comparative Example 11 | Comparative Example 12 |
|---|---|---|---|---|---|---|---|---|---|---|---|---|---|---|
| | Component(A) | a-1 | --- | --- | 30.0 | --- | --- | --- | --- | --- | --- | 55.8 | 45.0 | 34.8 |
| | | a-2 | 45.0 | --- | --- | --- | --- | --- | --- | --- | --- | --- | --- | --- |
| | | a-3 | --- | --- | --- | --- | --- | --- | --- | --- | --- | --- | --- | --- |
| | | a-4 | --- | --- | --- | --- | --- | --- | --- | --- | --- | --- | --- | --- |
| | | a-5 | --- | 36.8 | --- | 36.0 | 18.2 | 36.7 | 14.7 | --- | --- | --- | --- | --- |
| | | a-6 | --- | --- | --- | --- | 18.2 | --- | --- | 25.0 | 22.85 | --- | --- | --- |
| | | a-7 | --- | --- | --- | --- | --- | --- | 22.0 | 12.0 | 22.85 | --- | --- | --- |
| | | a-8 | --- | --- | --- | --- | --- | --- | --- | --- | --- | --- | --- | --- |
| | | a-9 | --- | --- | --- | --- | --- | --- | --- | --- | --- | --- | --- | --- |
| | Component(B) | b-1 | 10.0 | 8.0 | --- | --- | --- | --- | --- | --- | --- | --- | --- | --- |
| | | b-2 | --- | --- | --- | --- | --- | --- | --- | --- | --- | --- | --- | --- |
| | Component(X) | x-1 | --- | --- | --- | 10.0 | 9.1 | --- | --- | --- | --- | --- | --- | --- |
| | | x-2 | --- | --- | --- | --- | --- | 8.3 | 8.3 | 7.4 | 8.8 | 7.0 | 10.0 | 13.0 |
| | | x-3 | --- | --- | 27.0 | --- | --- | --- | --- | --- | --- | --- | --- | --- |
| | Component(C) | c-1 | 45.0 | 55.2 | 43.0 | 54.0 | 54.5 | 55.0 | 55.0 | 55.8 | 45.7 | 37.2 | 45.0 | 52.2 |
| | | c-2 | --- | --- | --- | --- | --- | --- | --- | --- | --- | --- | --- | --- |
| | Component(D) | d-1 | --- | --- | --- | --- | --- | --- | --- | --- | --- | --- | --- | --- |

| | | Compara-tive Exam-ple 1 | Compara-tive Exam-ple 2 | Compara-tive Exam-ple 3 | Compara-tive Exam-ple 4 | Compara-tive Exam-ple 5 | Compara-tive Exam-ple 6 | Compara-tive Exam-ple 7 | Compara-tive Exam-ple 8 | Compara-tive Exam-ple 9 | Compara-tive Exam-ple 10 | Compara-tive Exam-ple 11 | Compara-tive Exam-ple 12 |
|---|---|---|---|---|---|---|---|---|---|---|---|---|---|
| Molecular weight of peak top of component (A) | Other peak | --- | --- | --- | --- | --- | --- | --- | --- | --- | --- | --- | --- |
| | | --- | 127,000 | --- | 127,000 | 127,000 | 127,000 | 127,000 | --- | --- | --- | --- | --- |
| | Peak Lp | 235,000 | --- | 177,000 | --- | --- | --- | 168,000 | 168,000 | 168,000 | 177,000 | 177,000 | 177,000 |
| | | --- | 358,000 | --- | 358,000 | 358,000 | 358,000 | 358,000 | --- | --- | --- | --- | --- |
| | Peak Hp | --- | --- | 499.000 | --- | 408,000 | --- | --- | 408,000 | 408,000 | 499.000 | 499.000 | 499.000 |
| (A)/((A)+(C))※ | | 50 | 40 | 40 | 40 | 40 | 40 | 40 | 40 | 50 | 60 | 50 | 40 |
| Evaluation results | MFR 230°C, 2.18kg F (g/10min.) | --- | 2 | --- | 8 | 2 | 2 | 3 | 0.5 | --- | | | |
| | MFR 230°C, 5kgf (g/10min.) | 9 | 61 | 8 | --- | --- | 43 | --- | 13 | 18 | 0.1 | 1.6 | 58 |
| | Comression set 70°C. 22h (%) | 39 | 39 | 44 | 48 | 47 | 48 | 49 | 44 | 40 | 42 | 39 | 41 |
| | Duro hardness A 15s (-) | 53 | 30 | 82 | 40 | 48 | 35 | 36 | 41 | 36 | 32 | 32 | 30 |

※Content of Component(A) relative to the total of component (A) and component (c) (% by mass)

EP 4 389 823 A1

EP 4 389 823 A1

[Consideration]

The following can be seen from Tables 1 and 2.

The thermoplastic elastomer compositions of Examples 1 to 12 had good evaluation results for both compression set and moldability.

Comparative Example 1 was a thermoplastic elastomer composition obtained in the same manner as Example 1 except that a-2 having only a peak corresponding to peak Lp with respect to the component (A) was used, and had a high compression set.

Comparative Example 2 was a thermoplastic elastomer composition obtained using a-5, which had two peaks corresponding to peak Lp for the component (A), and had a high compression set.

Comparative Example 3 was a thermoplastic elastomer composition obtained by using high density polyethylene of other resin x-3 instead of the component (B), and had a high compression set.

Comparative Example 4 was a thermoplastic elastomer composition obtained by using a-5 having two peaks corresponding to peak Lp with respect to the component (A) and propylene homopolymer of other resin x-1 instead of the component (B), and had a high compression set.

Comparative Example 5 was a thermoplastic elastomer composition obtained by using propylene homopolymer of other resin x-1 instead of the component (B), and had a high compression set.

Comparative Example 6 was a thermoplastic elastomer composition obtained by using a-5 having two peaks corresponding to peak Lp with respect to the component (A) and propylene homopolymer of other resin x-2 instead of the component (B), and had a high compression set.

Comparative Example 7 was a thermoplastic elastomer composition obtained by using a-5 and a-7 having three peaks corresponding to peak Lp with respect to the component (A) and propylene homopolymer of other resin x-2 instead of the component (B), and had high compression set.

Comparative Examples 8 and 9 were thermoplastic elastomer compositions obtained by using a-6 and a-7 with respect to the component (A) and propylene homopolymer of other resin x-2 instead of the component (B), and had high compression set.

Comparative Examples 10 to 12 were thermoplastic elastomer compositions obtained in the same manner as Example 1, except that propylene homopolymer of other resin x-2 was used instead of the component (B), and had high compression set.

Although the present invention has been described in detail using specific embodiments, it will be apparent to those skilled in the art that various changes can be made without departing from the spirit and scope of the present invention.

This application is based on Japanese Patent Application No. 2021-134245 filed on August 19, 2021, which is incorporated by reference in its entirety.

[Explanation of symbols]

10, 20, 30, 40, 50: Rubber stopper
10A, 20A, 30A, 40A, 50A: Base body
10a, 20a, 30a, 40a, 50a: Base part (leg part)
10b, 20b, 30b, 40b, 50b: Lid pant
11, 12, 13, 14, 21, 22, 23, 31, 32, 33, 41, 51: Needle pricking part

**Claims**

1. A thermoplastic elastomer composition comprising the following component (A) and component (B).

   Component (A): a styrene-based thermoplastic elastomer having the following molecular weight distribution. The styrene-based thermoplastic elastomer has a peak top in the range of 360,000 or more and 600,000 or less in a differential molecular weight distribution diagram calculated in terms of styrene obtained by gel permeation chromatographic analysis of the styrene thermoplastic elastomer.
   Component (B): hydrocarbon group-grafted polypropylene

2. The thermoplastic elastomer composition according to claim 1, wherein the component (A) is a styrene-based thermoplastic elastomer having the following molecular weight distribution.

The styrene-based thermoplastic elastomer has a peak top in the range of 400,000 or more and 600,000 or less in a differential molecular weight distribution diagram calculated in terms of styrene obtained by gel permeation chromatographic analysis of the styrene thermoplastic elastomer.

3. The thermoplastic elastomer composition according to claim 2, wherein the component (A) is a styrene-based thermoplastic elastomer having the following molecular weight distribution.
The styrene-based thermoplastic elastomer has a peak Lp having a peak top in the range of 100,000 or more and 400,000 or less and a peak Hp having a peak top in the range more than 400,000 and 600,000 or less in a differential molecular weight distribution diagram calculated in terms of styrene obtained by gel permeation chromatographic analysis of the styrene thermoplastic elastomer.

4. The thermoplastic elastomer composition according to any one of claims 1 to 3, further comprising the following component (C).
Component (C): hydrocarbon softener for rubber

5. The thermoplastic elastomer composition according to claim 4, wherein the content of the component (B) is 3% by mass or more and 30% by mass or less in a total of 100% by mass of the components (A), (B) and (C).

6. The thermoplastic elastomer composition according to claim 4, wherein the content of the component (A) is 35% by mass or more and 65% by mass or less in a total of 100% by mass of the components (A) and (C).

7. The thermoplastic elastomer composition according to claim 5, wherein the content of the component (A) is 35% by mass or more and 65% by mass or less in a total of 100% by mass of the components (A) and (C).

8. The thermoplastic elastomer composition according to any one of claims 1 to 3, having a compression set of 40% or less.

9. The thermoplastic elastomer composition according to claim 4, having a compression set of 40% or less.

10. The thermoplastic elastomer composition according to claim 5, having a compression set of 40% or less.

11. The thermoplastic elastomer composition according to claim 6, having a compression set of 40% or less.

12. The thermoplastic elastomer composition according to claim 7, having a compression set of 40% or less.

13. The thermoplastic elastomer composition according to any one of claims 1 to 3, having a melt flow rate (230°C, load 5 kgf) of 0.5 g/10 minutes or more and 200 g/10 minutes or less.

14. The thermoplastic elastomer composition according to any one of claims 1 to 3, having a Duro hardness A of 10 or more and 50 or less.

15. The thermoplastic elastomer composition according to claim 4, having a Duro hardness A of 10 or more and 50 or less.

16. The thermoplastic elastomer composition according to claim 5, having a Duro hardness A of 10 or more and 50 or less.

17. The thermoplastic elastomer composition according to claim 6, having a Duro hardness A of 10 or more and 50 or less.

18. The thermoplastic elastomer composition according to claim 7, having a Duro hardness A of 10 or more and 50 or less.

19. The thermoplastic elastomer composition according to claim 8, having a Duro hardness A of 10 or more and 50 or less.

20. The thermoplastic elastomer composition according to any one of claims 1 to 3, further comprising the following component (D).
Component (D): polyphenylene ether-based resin composition

21. A molded article made of the thermoplastic elastomer composition according to any one of claims 1 to 3.

22. A molded article made of the thermoplastic elastomer composition according to claim 4.

EP 4 389 823 A1

23. A molded article made of the thermoplastic elastomer composition according to claim 5.

24. A molded article made of the thermoplastic elastomer composition according to claim 6.

25. A molded article made of the thermoplastic elastomer composition according to claim 7.

26. A molded article made of the thermoplastic elastomer composition according to claim 8.

27. A molded article made of the thermoplastic elastomer composition according to claim 9.

28. A molded article made of the thermoplastic elastomer composition according to claim 10.

29. A molded article made of the thermoplastic elastomer composition according to claim 11.

30. A molded article made of the thermoplastic elastomer composition according to claim 12.

31. A molded article made of the thermoplastic elastomer composition according to claim 13.

32. A molded article made of the thermoplastic elastomer composition according to claim 14.

33. The molded article according to claim 21, wherein the molded article is a rubber stopper having a needle pricking part.

34. The molded article according to claim 22, wherein the molded article is a rubber stopper having a needle pricking part.

35. The molded article according to claim 23, wherein the molded article is a rubber stopper having a needle pricking part.

36. The molded article according to claim 24, wherein the molded article is a rubber stopper having a needle pricking part.

37. The molded article according to claim 25, wherein the molded article is a rubber stopper having a needle pricking part.

38. The molded article according to claim 26, wherein the molded article is a rubber stopper having a needle pricking part.

39. The molded article according to claim 27, wherein the molded article is a rubber stopper having a needle pricking part.

40. The molded article according to claim 28, which is a rubber plug having a needle prick.

41. The molded article according to claim 29, wherein the molded article is a rubber stopper having a needle pricking part.

42. The molded article according to claim 30, wherein the molded article is a rubber stopper having a needle pricking part.

43. The molded article according to claim 31, wherein the molded article is a rubber stopper having a needle pricking part.

44. The molded article according to claim 32, wherein the molded article is a rubber stopper having a needle pricking part.

[Fig. 1]

(a)

(b)

[Fig. 2]

(a)

(b)

[Fig. 3]

[Fig. 4]

[Fig. 5]

(a)

51
50
B - - - B
50A(50b)

(b)

51
50
50b
50a
50A
50c

## INTERNATIONAL SEARCH REPORT

| International application No. |
|---|
| **PCT/JP2022/030123** |

**A. CLASSIFICATION OF SUBJECT MATTER**

*C08L 53/02*(2006.01)i; *C08L 51/06*(2006.01)i; *C08L 71/12*(2006.01)i
FI:   C08L53/02; C08L51/06; C08L71/12

According to International Patent Classification (IPC) or to both national classification and IPC

**B. FIELDS SEARCHED**

Minimum documentation searched (classification system followed by classification symbols)

   C08L53/02; C08L51/06; C08L71/12

Documentation searched other than minimum documentation to the extent that such documents are included in the fields searched

   Published examined utility model applications of Japan 1922-1996
   Published unexamined utility model applications of Japan 1971-2022
   Registered utility model specifications of Japan 1996-2022
   Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2015-98542 A (MITSUBISHI CHEMICALS CORPORATION) 28 May 2015 (2015-05-28) claims, paragraphs [0023], [0027], [0058]-[0075], examples | 1-32 |
| A | | 33-44 |
| A | JP 2010-275459 A (BRIDGESTONE CORPORATION) 09 December 2010 (2010-12-09) entire text, particularly, examples | 1-44 |
| A | JP 2005-75896 A (RIKEN TECHNOS CORPORATION) 24 March 2005 (2005-03-24) entire text, particularly, examples | 1-44 |
| A | WO 2017/150714 A1 (MCPP INNOVATION LIMITED LIABILITY COMPANY) 08 September 2017 (2017-09-08) claims, particularly, examples | 1-44 |
| A | JP 2019-131671 A (MCPP INNOVATION LIMITED LIABILITY COMPANY) 08 August 2019 (2019-08-08) claims, particularly, examples | 1-44 |

☑ Further documents are listed in the continuation of Box C.   ☑ See patent family annex.

| \* | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **07 September 2022** | **27 September 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| | International application No. |
|---|---|
| | **PCT/JP2022/030123** |

**C.    DOCUMENTS CONSIDERED TO BE RELEVANT**

| Category* | Citation of document, with indication, where appropriate, of the relevant passages | Relevant to claim No. |
|---|---|---|
| A | JP 2019-44113 A (MCPP INNOVATION LIMITED LIABILITY COMPANY) 22 March 2019 (2019-03-22)<br>claims, examples | 1-44 |
| A | US 2014/0094538 A1 (POLYONE CORPORATION) 03 April 2014 (2014-04-03)<br>claims, examples | 1-44 |

Form PCT/ISA/210 (second sheet) (January 2015)

**INTERNATIONAL SEARCH REPORT**
Information on patent family members

International application No.

**PCT/JP2022/030123**

| Patent document cited in search report | | | Publication date (day/month/year) | Patent family member(s) | | | Publication date (day/month/year) |
|---|---|---|---|---|---|---|---|
| JP | 2015-98542 | A | 28 May 2015 | (Family: none) | | | |
| JP | 2010-275459 | A | 09 December 2010 | (Family: none) | | | |
| JP | 2005-75896 | A | 24 March 2005 | (Family: none) | | | |
| WO | 2017/150714 | A1 | 08 September 2017 | US | 2018/0371250 | A1 | |
| | | | | claims, examples | | | |
| | | | | EP | 3425003 | A1 | |
| | | | | CN | 108779320 | A | |
| JP | 2019-131671 | A | 08 August 2019 | (Family: none) | | | |
| JP | 2019-44113 | A | 22 March 2019 | (Family: none) | | | |
| US | 2014/0094538 | A1 | 03 April 2014 | WO | 2012/166779 | A2 | |
| | | | | claims, examples | | | |
| | | | | EP | 2714798 | A2 | |
| | | | | CA | 2837325 | A1 | |
| | | | | CN | 103562302 | A | |
| | | | | KR | 10-2014-0017683 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017150714 A1
- JP 5703990 B
- JP 2015098542 A
- JP 2021134245 A